(19) Europäisches Patentamt
European Patent Office
Office européen des brevets

(11) EP 2 271 744 B1

(12) **FASCICULE DE BREVET EUROPEEN**

(45) Date de publication et mention de la délivrance du brevet:
**20.03.2013 Bulletin 2013/12**

(21) Numéro de dépôt: **09734424.6**

(22) Date de dépôt: **16.04.2009**

(51) Int Cl.:
***C12N 1/20*** (2006.01) ***A23C 9/123*** (2006.01)
***C12R 1/225*** (2006.01)

(86) Numéro de dépôt international:
**PCT/FR2009/000443**

(87) Numéro de publication internationale:
**WO 2009/130423 (29.10.2009 Gazette 2009/44)**

(54) **NOUVELLE SOUCHE DE LACTOBACILLUS PARACASEI SUBSP. PARACASEI DOTEE DE PROPRIETES ANTIMICROBIENNES ET IMMUNOMODULATRICES**

NEUER STAMM DES LACTOBACILLUS PARACASEI SUBSPECIES PARACASEI MIT ANTIMIKROBIELLEN UND IMMUNMODULIERENDEN EIGENSCHAFTEN

NOVEL STRAIN OF LACTOBACILLUS PARACASEI SUBSPECIES PARACASEI HAVING ANTIMICROBIAL AND IMMUNOMODULATORY PROPERTIES

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **18.04.2008 FR 0802158**

(43) Date de publication de la demande:
**12.01.2011 Bulletin 2011/02**

(73) Titulaire: **Compagnie Gervais Danone**
**75009 Paris (FR)**

(72) Inventeurs:
- **CHAMBAUD, Isabelle**
  **F-92130 Issy Les Moulineaux (FR)**
- **KHLEBNIKOV, Artem**
  **New York 10804 (US)**
- **VILLAIN, Anne-Catherine**
  **F-92160 Juvisy Sur Orge (FR)**
- **GROMPONE, Gianfranco**
  **F-75005 Paris (FR)**
- **SAINT DENIS, Thierry**
  **F-94300 Vincennes (FR)**
- **DRUESNE, Anne**
  **F-91190 Villiers Le Bacle (FR)**
- **SMOKVINA, Tamara**
  **F-91400 Orsay (FR)**

(74) Mandataire: **Rançon, Xavier Lucien Abel et al**
**Cabinet Ores**
**36 Rue de Saint Petersbourg**
**75008 Paris (FR)**

(56) Documents cités:
**WO-A-96/20607** **WO-A-02/053706**
**WO-A-2008/047391**

- **LJUNGH ASA ET AL: "Isolation, selection and characteristics of Lactobacillus paracasei subsp. paracasei F19" MICROBIAL ECOLOGY IN HEALTH AND DISEASE, vol. 14, no. Supplement 3, 2002, pages 4-6, XP009105487 ISSN: 0891-060X**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

SEQUENCE LISTING

<110> COMPAGNIE GERVAIS DANONE CHAMBAUD, Isabelle KHLEBNIKOV, Artem VILLAIN, Anne-Catherine GROMPONE, Gianfranco SAINT DENIS, Thierry DRUESNE, Anne SMOKVINA, Tamara

<120> NOUVELLE SOUCHE DE LACTOBACILLUS PARACASEI SUBSP. PARACASEI DOTEE DE PROPRIETES ANTIMICROBIENNES ET IMMUNOMODULATRICES

<130> F191PCT233

<160> 4

<170> PatentIn version 3.3

<210> 1
<211> 3323
<212> DNA
<213> Lactobacillus paracasei

<400> 1

```
gttttccccg cacatgcggg ggtgatcctt acaagctgaa tggtacgcca cagccagcgc     60
tgttttcccc gcacatgcgg gggtgatccc tacatcaacg aaacgctgat aacgcgatca    120
gcgttttccc cgcacatgcg ggggtgatcc tgagatcagg gtttatccaa ctaccgtctt    180
caagttttcc ccgcacatgc gggggtgatc cctgatctca aagccttatc cagcatcaga    240
tattgttttc cccgcacatg cgggggtgat cctggatcag gcaaaaggcg atcttgataa    300
ctacagtttt ccccgcacat gcgggggtga tccctgttta gcctaatcat taagcgcctc    360
aacgttgttt tccccgcaca tgcgggggtg atccctgtat gaacccatta cgcttgccat    420
gcttgctgtt ttccccgcac atgcgggggt gatcctatca agcccgatta tttggttcac    480
gattctaagt tttccccgca catgcggggg tgatccttat gtcattagga acgaatgcct    540
atcaacatag ttttccccgc acatgcgggg gtgatcctaa ccgtttagaa gggatcacta    600
gccagctgaa gttttccccg cacatgcggg ggtgatcctg taaatgtccg cgtccgatgg    660
ttcgtccgcc ggttttcccc gcacatgcgg gggtgatcct ggtaaattgc aggctcaata    720
tacccagcta aagttttccc cgcacatgcg ggggtgatcc cgccccctcc cgcataacct    780
gtgttgtcta aacgttttcc ccgcacatgc gggggtgatc caaaccaaga gcaacaaaat    840
gttattaagc cattgttttc cccgcacatg cgggggtgat cctacgctca atctgcagct    900
tgctggtatt gttcagtttt ccccgcacat gcgggggtga tcccttggtt atcaagcgac    960
acagacccca caaacagttt tccccgcaca tgcgggggtg atcccagaat aatgttctta   1020
aagctgtcct tggtgaggtt ttccccgcac atgcgggggt gatcccgcgg ccgtaacagc   1080
ggcagaacag cttggtgtgt tttccccgca catgcggggg tgatcctaag gtgagtgcat   1140
atgtctaaaa aaattgatcg ttttccccgc acatgcgggg gtgatcctat ggcagctaat   1200
acggatgcca ttttagctgg gttttccccg cacatgcggg ggtgatcccg gactgctagt   1260
```

agtatcaaaa tacatgatga ggttttcccc gcacatgcgg gggtgatccc gacaagttcg 1320
gaatttctca gattggcggt tagttttccc cgcacatgcg gggtgatcc ccaagataaa 1380
tctatatctt tgcaataaat agcgttttcc ccgcacatgc gggggtgatc ccggtggtat 1440
cccaaacttt tgacataccc tttggttttc cccgcacatg cgggggtgat cctaaatgtt 1500
cccttaatcc tcaaaaggcc tttcggtttt ccccgcacat gcgggggtga tccccaatga 1560
cattattttt ataatcagtc atttctgttt tccccgcaca tgcgggggtg atcccaaaat 1620
gccgaaggtc aacaaaaatg gtgatccgtt ttccccgcac atgcgggggt gatcccaatg 1680
catcaaacac catttcgaag ggcattaggt tttccccgca catgcggggg tgatcccaac 1740
agatgatgtc aaaatcccag caagtgattg ttttccccgc acatgcgggg gtgatccccg 1800
ctgaaatatc gctaatcatg ccatcaatga gttttccccg cacatgcggg ggtgatcccc 1860
attcaggaga tctcgaatac gcttttcttt agttttcccc gcacatgcgg gggtgatccc 1920
cagccactgg taaacctgat cggtgttgat tagttttccc cgcacatgcg gggtgatcc 1980
tggtcattga tgattaaaaa caagccaatg gcagttttcc ccgcacatgc gggggtgatc 2040
ctaactggct caacaggata agtgccacca ttatgttttc cccgcacatg cgggggtgat 2100
cccatcgtct tctgtataag aagacggatc agatggtttt ccccgcacat gcgggggtga 2160
tccttacttg tccgactaat ggactgactg agaatggttt tccccgcaca tgcgggggtg 2220
atcctaaagc tggttacaag gtaccggcca acatggggtt ttccccgcac atgcgggggt 2280
gatcctacca taggttggct gggtttaacg caatgtgagt tttccccgca catgcggggg 2340
tgatcccaac aatttacaaa acgccattca agaaagttag ttttccccgc acatgcgggg 2400
gtgatcctca gacttagccg aatccacctt cttgtctttg gttttccccg cacatgcggg 2460
ggtgatcccc agtcagagta atgtgggggc cagacgaaat agttttcccc gcacatgcgg 2520
gggtgatccc gagatcattc aacacatata ttcacctcca cagttttccc cgcacatgcg 2580
ggggtgatcc tactagtcca tatcagctgg agatcagcgc ctagttttcc ccgcacatgc 2640
gggggtgatc ccacctaaag atgtggaagc atgaccatgg agacgttttc cccgcacatg 2700
cgggggtgat cctggcaagt gacgctaaag gatcacacca atactgtttt ccccgcacat 2760
gcgggggtga tcccagtagt tactatgcgt caaacaagca gattttgttt tccccgcaca 2820
tgcgggggtg atccctacaa agacaagtac gcgtttgctc gcaagatgtt ttccccgcac 2880
atgcgggggt gatcctacta agggagacgg cggtaagtat ggacatttgg ttttccccgc 2940
acatgcgggg gtgatcctaa caacattaaa ggtgaatgcc gccactgcaa gttttccccg 3000
cacatgcggg ggtgatcctg gtcaagctcg gcaagatttc gccagcgtgg ggttttcccc 3060
gcacatgcgg gggtgatccc aatcggtgca cgtccggctg ttggtaagtc gggttttccc 3120
cgcacatgcg gggtgatcc ttgtcgccat tctttgacgg agtttgaccc tgagttttcc 3180
gcgcacatgc gggggtgatc ccatggctag cgatggtatt atttctgggg ctgagttttc 3240
cgcgcacatg cgggggtgat cctatcagcc ggaagcggtg aagctggcgc gtttggtttt 3300

ccccgcacat gcgggggtga tcc 3323

<210> 2
<211> 28
<212> DNA
<213> Lactobacillus paracasei

<400> 2
gttttccccg cacatgcggg ggtgatcc 28

<210> 3
<211> 20
<212> DNA
<213> Lactobacillus paracasei

<400> 3
ctcaacagga taagtgccac 20

<210> 4
<211> 19
<212> DNA
<213> Lactobacillus paracasei

<400> 4
ggttggctgg gtttaacgc 19

## Description

L'invention est relative à une nouvelle souche de *Lactobacillus paracasei subsp. paracasei* dotée de propriétés antimicrobiennes et immunomodulatrices.

De très nombreuses études scientifiques ont rapporté les effets bénéfiques sur la santé de certains microorganismes présents dans des aliments fermentés, notamment des produits laitiers. Ces microorganismes sont communément dénommés «probiotiques». Selon la définition généralement admise à l'heure actuelle, les probiotiques sont « des microorganismes vivants, qui lorsqu'ils sont consommés en quantités adéquates, ont un effet bénéfique sur la santé de l'hôte » (Rapport FAO/OMS sur l'évaluation des propriétés sanitaires et nutritionnelles des probiotiques dans les aliments, y compris le lait en poudre contenant des bactéries lactiques vivantes ; Cordoba, Argentine ; 1-4 octobre 2001).

Il a été montré que la consommation de produits alimentaires contenant des bactéries probiotiques peut produire des effets favorables sur la santé, par l'intermédiaire notamment du rééquilibrage de la flore intestinale, de l'amélioration de la résistance aux infections, et de la modulation de la réponse immunitaire.

Les microorganismes probiotiques utilisés en alimentation humaine sont généralement des bactéries lactiques, appartenant principalement aux genres *Lactobacillus* et *Bifidobacterium,* et notamment à l'espèce *Lactobacillus paracasei subsp. paracasei* (dont une souche est décrite dans la Demande de brevet EP0794707).

Les effets bénéfiques sur la santé ne sont toutefois généralement pas communs à l'ensemble des bactéries d'un même genre, ni même d'une même espèce. Ils ne se rencontrent, le plus souvent, que chez certaines souches ; en outre, les effets observés peuvent varier qualitativement et/ou quantitativement d'une souche probiotique à l'autre, y compris à l'intérieur d'une même espèce.

Pour qu'un microorganisme puisse être considéré comme étant potentiellement utilisable comme probiotique, il doit satisfaire à au moins un, et idéalement plusieurs, des critères suivants :

- présenter une activité inhibitrice vis-à-vis de microorganismes pathogènes pouvant être présents dans la flore intestinale, cette activité pouvant résulter soit de la capacité à adhérer aux cellules intestinales, excluant ou réduisant ainsi l'adhérence des pathogènes, soit de la capacité à produire des substances inhibant les pathogènes, soit de la combinaison de ces deux caractéristiques ;
- présenter des propriétés immunomodulatrices, et notamment immunostimulantes et/ou anti-inflammatoires.

En outre, si ce microorganisme est destiné à être incorporé dans un produit laitier, il doit de préférence présenter une croissance satisfaisante sur lait.

Enfin, il doit conserver une bonne viabilité, au cours de la production et de la conservation de l'aliment dans lequel il est incorporé, ainsi qu'après l'ingestion de cet aliment par le consommateur, de manière à pouvoir atteindre l'intestin et à survivre dans le milieu intestinal.

Il est toutefois à noter que bien que la viabilité soit essentielle pour répondre à la définition actuelle de « probiotique », il a été montré que certains des effets bénéfiques associés à des souches probiotiques pouvaient être obtenus même en l'absence de bactéries vivantes, et étaient attribuables à certaines fractions bactériennes ou à des fractions actives de leurs surnageants de culture. Par exemple, la Demande PCT WO2004093898 décrit une préparation immunomodulatrice obtenue par fractionnement du surnageant de culture de la souche CNCM I-2219.

Les Inventeurs sont maintenant parvenus à isoler une nouvelle souche de *Lactobacillus paracasei subsp. paracasei,* et répondant aux critères indiqués ci-dessus.

La présente invention a pour objet cette souche, qui a été déposée selon le Traité de Budapest, auprès de la CNCM (Collection Nationale de Cultures de Microorganismes, 25 rue du Docteur Roux, Paris), le 9 novembre 2006, sous le numéro I-3689.

La souche CNCM I-3689 possède les caractéristiques suivantes:

- Morphologie : microorganisme Gram-positif, petits bacilles fins, isolés ou petites chaines.
- Fermentation des sucres suivants (résultats obtenus sur une galerie api 50 CH - Milieu MRS API à 37°C pendant 48h): Ribose, galactose, D-glucose, D-fructose, D-mannose, mannitol, N-acétylglucosamine, arbutine, cellobiose, maltose, lactose, trehalose, melezitose, D-turanose, D-tagatose, gluconate.
- Présence d'un seul locus CRISPR de séquence SEQ ID NO : 1, contenant une séquence répétée représentée par la séquence nucléotidique SEQ ID NO : 2.

Elle possède d'autre part des propriétés anti-microbiennes, se traduisant par une forte capacité à inhiber la croissance en culture de microorganismes pathogènes, notamment *Escherichia coli, Salmonella enteritidis* et *Listeria monocytogenes.*

La souche CNCM I-3689 possède également des propriétés immunomodulatrices, et notamment anti-inflammatoires.

La présente description englobe également pour objet des souches de *Lactobacillus paracasei subsp. paracasei* susceptibles d'être obtenues par mutagénèse ou par transformation génétique de la souche CNCM I-3689. De préférence, ces souches conservent les propriétés anti-microbiennes et immunomodulatrices de la souche CNCM I-3689. Il peut s'agir de souches dans lesquelles un ou plusieurs des gènes endogènes de la souche CNCM I-3689 a (ont) été muté(s), par exemple de manière à modifier certaines de ses propriétés métaboliques (e.g. la capacité de cette souche à métaboliser des sucres, sa résistance au transit intestinal, sa résistance à l'acidité, sa post acidification ou sa production de métabolites). Il peut s'agir également de souches résultant de la transformation génétique de la souche CNCM I-3689 par un ou plusieurs gène(s) d'intérêt, permettant par exemple de conférer à ladite souche des caractéristiques physiologiques supplémentaires, ou d'exprimer des protéines d'intérêt thérapeutique ou vaccinal, que l'on souhaite administrer par l'intermédiaire de ladite souche.

Ces souches peuvent être obtenues à partir de la souche CNCM I-3689 par les techniques classiques de mutagénèse aléatoire ou dirigée et de transformation génétique de lactobacilles, telles que celles décrites par exemple par GURY et al. (Arch Microbiol., 182, 337-45, 2004) ou par VELEZ et al. (Appl Environ Microbiol., 73, 3595-3604, 2007), ou par la technique dénommée « genome shuffling » (PATNAIK et al. Nat Biotechnol, 20, 707-12, 2002; Wang Y. et al., J Biotechnol., 129, 510-15, 2007). Ces souches possèdent en particulier un locus CRISPR de séquence SEQ ID NO : 1.

La présente invention a également pour objet un procédé d'obtention d'une souche de *Lactobacillus paracasei subsp. paracasei* possédant des propriétés anti-microbiennes et/ou immunomodulatrices, comprenant une étape de mutagenèse ou de transformation génétique de la souche CNCM I-3689.

La présente invention a également pour objet un procédé d'obtention d'une fraction cellulaire possédant des propriétés anti-microbiennes et/ou immunomodulatrices à partir d'une souche de *Lactobacillus paracasei subsp. paracasei* conforme à l'invention. Il s'agit notamment de préparations d'ADN ou de préparations de parois bactériennes obtenues à partir de cultures de ladite souche. Il peut s'agir également de surnageants de culture ou de fractions de ces surnageants.

La présente invention a également pour objet des compositions comprenant une souche de *Lactobacillus paracasei subsp. paracasei* conforme à l'invention, ou une fraction cellulaire obtenue à partir de celle-ci.

Ces compositions peuvent être notamment des ferments lactiques, associant une souche de *Lactobacillus paracasei subsp. paracasei* conforme à l'invention avec une ou plusieurs autre(s) souche(s) de bactéries lactiques, probiotique(s) ou non. A titre d'exemple de souches de bactéries lactiques, on peut citer les souches *Lactobacillus bulgaricus* et *Streptococcus thermophilus.*

Il peut s'agir également de produits alimentaires, et notamment de produits laitiers, ou de produits pharmaceutiques ou cosmétiques comprenant une souche de *Lactobacillus paracasei subsp. paracasei* conforme à l'invention, ou une fraction cellulaire obtenue à partir de celle-ci.

Lorsque ladite souche est présente sous forme de bactéries vivantes, celles-ci seront de préférence présentes à raison d'au moins $10^5$ ufc par gramme, avantageusement au moins $10^6$ ufc par gramme de produit, plus avantageusement au moins $10^7$ ufc par gramme et encore plus avantageusement au moins $10^8$ ufc par gramme.

La présente invention sera mieux comprise à l'aide du complément de description qui va suivre qui se réfère à des exemples illustrant les propriétés anti-microbiennes, immunomodulatrices et anti-infectieuses de la souche CNCM I-3689, ainsi que le typage moléculaire de cette souche.

## EXEMPLE 1 : COMPARAISON DES PROPRIETES DE LA SOUCHE CNCM I-3689 AVEC CELLES DE SOUCHES PROBIOTIQUES CONNUES

Les propriétés de la souche CNCM I-3689 ont été comparées avec celles de différentes souches de l'art antérieur, connues pour leurs propriétés probiotiques.

La liste de ces souches est donnée dans le Tableau I ci-dessous.

Tableau I

| Genre | Espèce | Dénomination(s) | Numéro de publication de Demandes de Brevet |
|---|---|---|---|
| *Lactobacillus* | *johnsonii* | NCC533 = La1 = CNCM I-1225 | EP0577903 |
| *Lactobacillus* | *acidophilus* | NCFM | WO2004032639 |
| *Lactobacillus* | *acidophilus* | La-5 | |
| *Lactobacillus* | *casei* | Shirota | |
| *Lactobacillus* | *paracasei* | CRL431 = ATCC 55544 | |

(suite)

| Genre | Espèce | Dénomination(s) | Numéro de publication de Demandes de Brevet |
|---|---|---|---|
| *Lactobacillus* | *rhamnosus* | LGG = ATCC 53103 | US4839281 |
| *Lactobacillus* | *rhamnosus* | HN001 = NM97/09514 | WO9910476 |
| *Lactobacillus* | *plantarum* | ATCC 14917= DSMZ 20174=WCFS1 | |
| *Lactobacillus* | *plantarum* | Probi 299v = DSM 9843 | WO9391823 |
| *Lactobacillus* | *reuteri* | DSMZ 20016 | |
| *Lactobacillus* | *reuteri* | Biogaia SD 2112 = ATCC55730 | WO2004034808 |
| *Bifidobacterium* | *breve* | ATCC 15700 | |
| *Bifidobacterium* | *breve* | BBC50 = CNCM I-2219 | EP1189517 |
| *Bifidobacterium* | *infantis* | ATCC 15697 | |
| *Bifidobacterium* | *longum* | BB536 | |
| *Bifidobacterium* | *longum* | NCC2705 = CNCM I-2618 | |
| *Bifidobacterium* | *animalis subsp. lactis* | BB12 = ATCC 27536 | |
| *Bifidobacterium* | *animalis subsp. lactis* | HN019 = NM97/01925 | WO9910476 |

**1- Activité antimicrobienne**

La recherche d'activités anti-microbiennes a été effectuée contre trois bactéries pathogènes cibles : *Escherichia coli* E1392-75-2A, *Salmonella enteritidis* NIZO B1241 et *Listeria monocytogenes* 4B. Les bactéries lactiques ont été mises en culture sur boîtes de Petri dans deux milieux différents: milieu Elliker (ELLIKER et al., J. Dairy Sci., 39, 1611-1612, 1956), et milieu TGV (Tryptone-Glucose-Meat Extract).

Les boîtes sont incubées à 37°C jusqu'à apparition de colonies bactériennes. Les cultures de *Bifidobacterium* ont été effectuées sous anaérobiose. Une couche d'agar contenant du milieu BHI (brain-heart infusion) et le pathogène est alors coulée à la surface des boîtes. Les boîtes sont incubées de nouveau à 37°C pendant 24h. Les diamètres des zones d'inhibition du pathogène sont ensuite mesurés autour de chaque colonie de bactéries lactiques. Le score 1 correspond à un diamètre entre 1 et 3 mm. Le score 2 correspond à un diamètre entre 4 et 6 mm. Le score 3 correspond à un diamètre supérieur à 6 mm. Chaque expérience a été menée trois fois indépendamment pour chaque souche.

Les scores obtenus sur les pathogènes cibles dans chaque expérience ont été additionnés, pour obtenir, pour chaque bactérie lactique, un score global d'activité anti-microbienne.

Les résultats sont donnés dans le Tableau II ci-après.

Ces résultats montrent que parmi les souches testées, la souche CNCM I-3689 est avec la souche ATCC 55544 celle qui possède l'activité anti-microbienne la plus élevée.

Tableau II

| Genre | Espèce | Référence | (1) *E. coli*/ Elliker | (2) *Listeria*/ Elliker | (3) *Listeria*/ TGV | (4) *Salmonella* /Elliker | (5) *Salmonella* /TGV | Score anti-pathogènes (1+2+3+4+5) |
|---|---|---|---|---|---|---|---|---|
| *Lactobacillus* | *paracasei subsp. paracasei* | DN 114121 = CNCM I-3689 | 3 | 3 | 2 | 3 | 2 | 13 |
| *Lactobacillus* | *paracasei* | CRL431=ATCC55544 | 2 | 3 | 3 | 3 | 2 | 13 |
| *Lactobacillus* | *rhamnosus* | LGG = ATCC 53103 | 2 | 1 | 2 | 3 | 3 | 11 |
| *Lactobacillus* | *plantarum* | ATCC 14917= DSMZ 20174=WCFS1 | 2 | 1 | 2 | 3 | 2 | 10 |
| *Lactobacillus* | *casei* | Shirota | 2 | 3 | 1 | 1 | 2 | 9 |
| *Lactobacillus* | *rhamnosus* | HN001 = NM97109514 | 1 | 1 | 2 | 3 | 2 | 9 |
| *Lactobacillus* | *johnsonii* | NCC533=La1=I-1225 | 1 | 1 | 1 | 2 | 1 | 6 |
| *Lactobacillus* | *plantarum* | Probi 299v = DSM 9843 | 1 | 1 | 1 | 1 | 1 | 5 |
| *Lactobacillus* | *acidophilus* | La-5 | 1 | 1 | 2 | 0 | 1 | 5 |
| *Lactobacillus* | *acidophilus* | NCFM | 1 | 1 | 1 | 0 | 1 | 4 |
| *Bifidobacterium* | *animalis subsp. lactis* | BB12 = ATCC 27536 | 1 | 0 | 0 | 0 | 2 | 3 |
| *Lactobacillus* | *reuteri* | Biogaia SD 2112 ATCC55730 | 0 | 1 | 0 | 1 | 0 | 2 |
| *Bifidobacterium* | *longum* | BB536 | 0 | 0 | 0 | 0 | 0 | 0 |
| *Bifidobacterium* | *animalis subsp. lactis* | HN019 = NM97/01925 | 0 | 0 | 0 | 0 | 0 | 0 |
| *Lactobacillus* | *reuteri* | DSMZ 20016 | 0 | 0 | 0 | 0 | 0 | 0 |
| *Bifidobacterium* | *infantis* | ATCC 15697 | 0 | 0 | 0 | 0 | 0 | 0 |
| *Bifidobacferium* | *breve* | BBC50 = 1-2219 | 0 | 0 | 0 | 0 | 0 | 0 |
| *Bifidobacterium* | *breve* | ATCC 15700 | 0 | 0 | 0 | 0 | 0 | 0 |

(suite)

| Genre | Espèce | Référence | (1) *E. coli*/ Elliker | (2) *Listeria*/ Elliker | (3) *Listeria*/ TGV | (4) *Salmonella* /Elliker | (5) *Salmonella* /TGV | Score anti-pathogènes (1+2+3+4+5) |
|---|---|---|---|---|---|---|---|---|
| *Bifidobacterium* | *longum* | NCC2705 = CNCM I-2618 | 0 | 0 | 0 | 0 | 0 | 0 |

**2 - Immunomodulation**

Les propriétés immunomodulatrices des différentes bactéries lactiques ont été évaluées par la mesure du ratio IL-10/IL-12.

Des prélèvements de sang humain, obtenus chez des sujets sains, ont été dilués à un ratio 1:2 avec du PBS-CA (GIBCO) et purifiés grâce à un gradient de Ficoll (GIBCO). Après une centrifugation à 400×g pendant 30 min à 20°C, les PBMC (« peripherical blood mononuclear cells ») ont été prélevés. Trois étapes de lavage ont ensuite été effectuées, puis les PBMC ont été resuspendus dans un milieu de culture RPMI (GIBCO) supplémenté avec 1% de sérum de veau foetal, 1% de L-glutamine (GIBCO) et 150 μg/ml de gentamycine (GIBCO). Les PBMC ont été comptés au microscope et ajustés à une concentration de $2\times10^6$ cellules/ml, puis distribués (en aliquots de lml) dans des plaques 24-puits de cultures cellulaires (Corning, Inc.).

Les souches de *Lactobacillus* ont été cultivées en milieu MRS (DE MAN et al., J. Appl. Bacteriol. 23, 130-135, 1960), et les souches de *Bifidobacterium* en milieu MRS supplémenté avec 0.03 % de L-cystéine (Sigma) sous anaérobiose. Toutes les souches ont été incubées à une température de 37°C. La croissance des bactéries a été arrêtée en phase stationnaire, et les bactéries ont ensuite été lavées et resuspendues à une concentration de 3 unités MacFarlan dans du PBS contenant 20% de glycérol.

Un volume de 10 μl de préparation bactérienne a été ajouté dans chaque puits des plaques contenant les PBMC (ratio bactéries:cellule 10:1). Les plaques ont été incubées pendant 24 h à 37°C sous une atmosphère contenant 5% de $CO_2$. Le surnageant a ensuite été aspiré, centrifugé à 2000 rpm et stocké à -20°C.

Les souches bactériennes contrôles, aux propriétés immunomodulatrices connues, ont été inclues dans le test. Du PBS 20 % glycérol, sans bactéries, a également été utilisé comme contrôle négatif. L'expérience a été menée pour chaque souche sur des PBMCs issus de trois donneurs différents.

L'expression des cytokines a été mesurée par des tests ELISA en utilisant des kits commerciaux (Pharmingen, BD Biosciences). Deux cytokines ont été étudiées : IL-10 et IL-12.

Pour chaque souche testée, la moyenne du ratio IL-10/IL-12 a été calculée. Ces résultats sont rassemblés dans le Tableau III suivant.

Tableau III

| Genre | Espèce | Référence | Ratio IL-10/IL-12 |
|---|---|---|---|
| ***Lactobacillus*** | ***paracasei subsp. paracasei*** | **DN 114121 = CNCM 1-3689** | **11,8** |
| *Lactobacillus* | *plantarum* | Probi 299v = DSM 9843 | 12,7 |
| *Lactobacillus* | *acidophilus* | La-5 | 23,5 |
| *Lactobacillus* | *acidophilus* | NCFM | 23,6 |
| *Lactobacillus* | *johnsonii* | NCC533=La1=I-1225 | 23,7 |
| *Lactobacillus* | *plantarum* | ATCC 14917= DSMZ 20174=WCFS1 | 25,0 |
| *Lactobacillus* | *reuteri* | Biogaia SD 2112 ATCC55730 | 25,4 |
| *Bifidobacterium* | *longum* | BB536 | 26,4 |
| *Bifidobacterium* | *animalis subsp. lactis* | HN019 = NM97/01925 | 28,7 |
| *Bifidobacterium* | *animalis subsp. lactis* | BB12 = ATCC 27536 | 32,4 |
| *Lactobacillus* | *rhamnosus* | LGG = ATCC 53103 | 34,5 |
| *Lactobacillus* | *reuteri* | DSMZ 20016 | 42,0 |
| *Bifidobacterium* | *infantis* | ATCC 15697 | 51,4 |
| *Bifidobacterium* | *breve* | BBC50 = 1-2219 | 58,6 |
| *Lactobacillus* | *casei* | Shirota | 67,6 |
| *Bifidobacterium* | *breve* | ATCC 15700 | 68,5 |
| *Bifidobacterium* | *longum* | NCC2705 = CNCM 1-2618 | 72,6 |
| *Lactobacillus* | *rhamnosus* | HN001 = NM97/09514 | 92,1 |
| *Lactobacillus* | *paracasei* | CRL431=ATCC55544 | 164,0 |

Ces résultats montrent que la souche CNCM I-3689 présente d'importantes propriétés anti-inflammatoires sur ce modèle. Aucune des souches de référence testées ne présente d'aussi bonnes propriétés.

La Figure 1 représente le ratio IL-10/IL-12 de chaque souche bactérienne en fonction de son score anti-pathogène (unité arbitraire) déterminés ci-dessus. Cette Figure montre à quel point la souche CNCM I-3689 se différencie par rapport aux autres souches testées.

**3- Survie au stress intestinal**

Un modèle in vitro reflétant les conditions de stress intestinal a été utilisé.

Des cultures de bactéries lactiques sont réalisées dans du lait supplémenté avec de l'extrait de levure. Les cultures sont incubées pendant 24 à 48h, selon les espèces (jusqu'à la phase stationnaire de culture).

Un jus intestinal artificiel composé de sels biliaires de porc (à 3.3 g/l) et de tampon carbonate $NaHCO_3$ (à 16,5 g/l) est réalisé. Le pH est ajusté à 6,3. 1 ml de ce jus intestinal est ajouté à 100 $\mu$l de culture bactérienne. Les cultures sont ensuite incubées 5 heures. Ensuite, les populations bactériennes avant et après le stress sont évaluées sur boîtes.

Les valeurs sont exprimées de la manière suivante : Stress intestinal = log (ufc5h/ufc0h).

ufcXmin étant la concentration en bactéries exprimée en Unités Formant Colonies (UFC) après X minutes d'incubation.

Pour le stress intestinal, la survie est bonne quand la valeur est supérieure à -0,5, moyennement bonne quand la valeur est comprise entre -0,5 et -1,5 et mauvaise quand la valeur est inférieure à -1,5.

Les résultats sont donnés dans le Tableau IV suivant.

Tableau IV

| Genre | Espèce | Référence | Stress intestinal |
|---|---|---|---|
| ***Lactobacillus*** | ***paracasei subsp. paracasei*** | **DN 114121 = CNCM I-3689** | **-0,40** |
| *Bifidobacterium* | *animalis subsp. lactis* | HN019 = NM97/01925 | 3,40 |
| *Bifidobacterium* | *infantis* | ATCC 15697 | 2,79 |
| *Lactobacillus* | *reuteri* | Biogaia SD 2112 ATCC55730 | 1,00 |
| *Lactobacillus* | *johnsonii* | NCC533=La1=I-1225 | 0,69 |
| *Bifidobacterium* | *animalis subsp. lactis* | BB12 = ATCC 27536 | 0,07 |
| *Lactobacillus* | *paracasei* | CRL431=ATCC55544 | 0,04 |
| *Lactobacillus* | *rhamnosus* | HN001 = NM97/09514 | 0,00 |
| *Lactobacillus* | *rhamnosus* | LGG = ATCC 53103 | -0,07 |
| *Bifidobacterium* | *breve* | ATCC 15700 | -0,14 |
| *Bifidobacterium* | *longum* | NCC2705 = CNCM 1-2618 | -0,22 |
| *Lactobacillus* | *acidophilus* | La-5 | -0,23 |
| *Lactobacillus* | *plantarum* | ATCC 14917= DSMZ 20174=WCFS1 | -0,79 |
| *Bifidobacterium* | *breve* | BBC50 = I-2219 | -0,91 |
| *Lactobacillus* | *plantarum* | Probi 299v = DSM 9843 | -1,04 |
| *Bifidobacterium* | *longum* | BB536 | -1,12 |
| *Lactobacillus* | *acidophilus* | NCFM | -1,45 |
| *Lactobacillus* | *casei* | Shirota | -1,50 |
| *Lactobacillus* | *reuteri* | DSMZ 20016 | -1,91 |

4- **Conclusion**

Les résultats illustrés dans les Tableaux II, III et IV ci-dessus montrent que, parmi les différentes souches testées, la souche CNCM I-3689 est la seule à présenter à la fois des propriétés anti-microbiennes et des propriétés anti-inflammatoires importantes, accompagnées en outre de bonnes propriétés de résistance au stress intestinal.

**EXEMPLE 2** : **CROISSANCE SUR LAIT DE LA SOUCHE CNCM I-3689**

Les propriétés de croissance sur lait de la souche CNCM I-3689 ont été testées en utilisant le protocole suivant :

- Un milieu constitué de lait écrémé reconstitué par de l'eau additionnée de poudre de lait écrémé a été ensemencé avec la souche CNCM I-3689 (5, 6x$10^6$ ufc/g ou 1, 1x$10^7$ ufc/g).
- L'activité fermentaire de cette souche, reliée à sa croissance, est mesurée en suivant en continu le pH du milieu de croissance.

Les résultats sont illustrés par le graphique présenté à la Figure 2.
Ces résultats montrent que la souche CNCM I-3689 est capable de croître efficacement sur du lait, et qu'elle peut donc être utilisée dans la fabrication de produits laitiers fermentés.

**EXEMPLE 3 : TYPAGE MOLECULAIRE DE LA SOUCHE CNCM I-3689**

De nombreux organismes procaryotes possèdent un ou plusieurs loci CRISPR - acronyme pour « *Clustered Regularly Interspaced Short Palindromic Repeats* » (Jansen et al., 2002, OMICS, Vol 6, Nb 1, 23-33). Un locus CRISPR est caractérisé par des séquences répétées (ou DR) non-contiguës, généralement de 21 à 37 paires de bases (pb), séparées par des séquences uniques, généralement de 20 à 40 paires de bases appelées séquences variables (« *spacers* »). D'une souche bactérienne à l'autre, il est possible d'observer des différences sur :

- le nombre de loci CRISPRs,
- la position des loci CRISPRs dans le génome,
- le nombre de séquences répétées, et/ou
- la nature et/ou la taille des séquences variables.

**1- Identification du locus CRISPR de la souche CNCM I-3689**

La souche CNCM I-3689 a été séquencée. L'analyse de son génome à l'aide de la suite de logiciels ERGO™ a permis d'identifier un seul locus CRISPR dans cette souche. Ce locus, d'une taille de 3323 pbs, se situe à 20 paires de bases en aval de l'ORF RDBK00370. Sa séquence d'ADN génomique est représentée par la séquence SEQ ID NO : 1. Il est composé d'une unité répétitive (GTTTTCCCCGCACATGCGGGGGTGATCC ; SEQ ID NO : 2) qui est identique à celle du locus CRISPR de la souche ATCC 334 *(Lactobacillus casei),* et de 54 séquences variables *(spacers).*

**2- Construction d'amorces pour amplification PCR, spécifiques de la souche CNCM I-3689**

Plusieurs couples d'amorces oligonucléotidiques ont été définis à partir des séquences variables du locus CRISPR de la souche CNCM I-3689. Ces amorces ont été testées par amplification PCR sur plusieurs souches bactériennes afin de vérifier leur spécificité pour la souche CNCM I-3689.
Un couple d'amorces a été retenu :

- amorce OFF2486 (CTCAACAGGATAAGTGCCAC ; SEQ ID NO : 3), située dans la 33ème séquence variable du locus CRISPR, aux positions 2049-2068 ; TM = 60°C ;
- amorce OFF2488 (GGTTGGCTGGGTTTAACGC ; SEQ ID NO : 4), située dans la 37ème séquence variable du locus CRISPR, aux positions 2093-2110 ; TM = 60°C.

Les conditions de PCR retenues sont les suivantes :

Mélange réactionnel :

| | |
|---|---|
| ADN : | 1 µl |
| dNTPs: | 4 µl |
| Tampon 10X: | 5 µl |
| Amorce OFF24 86 : | 0,3 µl |
| Amorce OFF24 88 : | 0, 3 µl |
| ADN polymérase Ex taq™ : | 0,2 µl |
| Eau : | 39,2 µl |

(suite)

Cycles :

| | | |
|---|---|---|
| 95°C | 5' | |
| 95°C | 30'' | x 30 |
| 61°C | 30'' | |
| 72°C | 45'' | |
| 72°C | 10' | |
| 10°C | | |

La taille attendue du produit de PCR à partir de la souche CNCM I-3689 est de 263 pbs.

Le couple d'amorces OFF2486/OFF2488 a été testé sur la souche CNCM I-3689 et sur 18 autres souches de *Lactobacillus casei* différentes, dont les souches CNCM I-1518 et ATCC 334. Les souches CNCM I-1518 et ATCC 334 représentaient les témoins négatifs car lesdites amorces ne peuvent pas s'hybrider à la séquence d'ADN génomique de ces souches dans les conditions de PCR décrites ci-dessus.

Les résultats sont illustrés par l'électrophorèse sur gel d'agarose des produits d'amplification PCR, présentée à la Figure 3, où « 121 » représente la souche CNCM I-3689, « 001 » représente la souche CNCM I-1518, « S3 » à « S18 » représentent respectivement 16 souches différentes de *Lactobacillus casei* et « SL » représente un marqueur de poids moléculaire (SmartLadder ; Eurogentec).

Ces résultats montrent que le couple d'amorces OFF2486/OFF2488 est bien spécifique de la souche CNCM I-3689, dans la mesure où il a été obtenu des produits d'amplification PCR de taille attendue (c'est-à-dire environ 260 pbs) uniquement pour cette souche.

**Revendications**

1. Souche de *Lactobacillus paracasei* subsp. *paracasei* possédant des propriétés anti-microbiennes et immunomodulatrices, **caractérisée en ce qu'**il s'agit de la souche déposée auprès de la CNCM sous le numéro I-3689.

2. Souche de *Lactobacillus paracasei subsp. paracasei* possédant des propriétés anti-microbiennes et immunomodulatrices, **caractérisée en ce qu'**elle est susceptible d'être obtenue à partir d'une souche selon la revendication 1 par mutagenèse ou transformation génétique, et **en ce qu'**elle possède les propriétés anti-microbiennes et immunomodulatrices de la souche CNCM I-3689 et/ou possède un locus CRISPR de séquence SEQ ID NO : 1.

3. Procédé d'obtention d'une souche de *Lactobacillus paracasei subsp. paracasei* possédant des propriétés anti-microbiennes et/ou immunomodulatrices, **caractérisée en ce qu'**il comprend une étape de mutagenèse ou de transformation génétique de la souche CNCM I-3689.

4. Procédé d'obtention d'une fraction cellulaire possédant des propriétés anti-microbiennes et/ou immunomodulatrices, **caractérisée en ce que** ladite fraction cellulaire est obtenue à partir d'une souche de *Lactobacillus paracasei subsp. paracasei* selon l'une quelconque des revendications 1 ou 2.

5. Procédé selon la revendication 4, **caractérisé en ce que** ladite fraction cellulaire est choisie dans le groupe constitué par : une préparation d'ADN, une préparation de parois bactériennes, un surnageant de culture et une fraction dudit surnageant.

6. Composition comprenant une souche de *Lactobacillus paracasei subsp. paracasei* selon l'une quelconque des revendications 1 ou 2, ou une fraction cellulaire obtenue selon le procédé défini à l'une quelconque des revendications 4 ou 5.

7. Composition selon la revendication 6, **caractérisée en ce qu'**il s'agit d'un produit alimentaire.

8. Composition selon la revendication 6, **caractérisée en ce qu'**il s'agit d'un produit pharmaceutique.

**9.** Composition selon la revendication 6, **caractérisée en ce qu'**il s'agit d'un produit cosmétique.

**10.** Utilisation d'une souche de *Lactobacillus paracasei subsp. paracasei* selon l'une quelconque des revendication 1 ou 2, ou une fraction cellulaire obtenue selon le procédé défini à l'une quelconque des revendications 4 ou 5 pour l'obtention d'une composition choisie dans le groupe constitué par un produit alimentaire, un produit pharmaceutique et un produit cosmétique.

**11.** Souche de *Lactobacillus paracasei* subsp. *paracasei* selon l'une quelconque des revendications 1 ou 2 pour utilisation comme agent immunomodulateur.

**12.** Souche de *Lactobacillus paracasei* subsp. *paracasei* selon l'une quelconque des revendications 1 ou 2 pour utilisation comme agent anti-microbien.

## Claims

**1.** A strain of *Lactobacillus paracasei* subsp. *paracasei* having anti-microbial and immunomodulating properties, **characterised in that** it is the strain filed with the CNCM under number I-3689.

**2.** A strain of *Lactobacillus paracasei* subsp. *paracasei* having anti-microbial and immunomodulating properties, **characterised in that** it can be obtained from a strain according to claim 1 by mutagenesis or genetic transformation, and **in that** it possesses the anti-microbial and immunomodulating properties of strain CNCM I-3689 and/or comprises a CRISPR locus having the sequence SEQ ID NO : 1.

**3.** A process for obtaining a strain of *Lactobacillus paracasei* subsp. *paracasei* having anti-microbial and/or immunomodulating properties, **characterised in that** it comprises a stage of mutagenesis or genetic transformation of strain CNCM I-3689.

**4.** A process for obtaining a cell fraction having anti-microbial and/or immunomodulating properties, **characterised in that** the cell fraction is obtained from a strain of *Lactobacillus paracasei* subsp. *paracasei* according to either of claims 1 or 2.

**5.** A process according to claim 4, **characterised in that** the cell fraction is selected from the group comprising a DNA preparation, a bacterial wall preparation, a culture supernatant and a fraction of the supernatant.

**6.** A composition comprising a strain of *Lactobacillus paracasei* subsp. *paracasei* according to either of claims 1 or 2, or a cell fraction obtained according to the process specified in either of claims 4 or 5.

**7.** A composition according to claim 6, **characterised in that** it is a food product.

**8.** A composition according to claim 6, **characterised in that** it is a pharmaceutical product.

**9.** A composition according to claim 6, **characterised in that** it is a cosmetic product.

**10.** Use of a strain of *Lactobacillus paracasei* subsp. *paracasei* according to either of claims 1 or 2, or a cell fraction obtained according to the process specified in either of claims 4 or 5 to obtain a composition selected from the group consisting of a food product, a pharmaceutical product and a cosmetic product.

**11.** A strain of *Lactobacillus paracasei* subsp. *paracasei* according to either of claims 1 or 2 for use as an immunomodulating agent.

**12.** A strain of *Lactobacillus paracasei* subsp. *paracasei* according to either of claims 1 or 2 for use as an anti-microbial agent.

## Patentansprüche

**1.** *Lactobacillus paracasei subsp. paracasei*-Stamm mit antimikrobiellen und immunmodulatorischen Eigenschaften, **dadurch gekennzeichnet, dass** es sich um den bei der CNCM unter der Nummer I-3689 hinterlegten Stamm handelt.

2. *Lactobacillus paracasei subsp. paracasei*-Stamm mit antimikrobiellen und immunmodulatorischen Eigenschaften, **dadurch gekennzeichnet, dass** er aus einem Stamm nach Anspruch 1 durch Mutagenese oder genetische Transformation erhältlich ist, und dadurch, dass er die antimikrobiellen und immunmodulatorischen Eigenschaften des Stamms CNCM I-3689 besitzt und/oder einen CRISPR-Lokus der Sequenz SEQ ID NO: 1 besitzt.

3. Verfahren zum Erhalt eines *Lactobacillus paracasei subsp. paracasei*-Stamms mit antimikrobiellen und/oder immunmodulatorischen Eigenschaften, **dadurch gekennzeichnet, dass** es einen Schritt der Mutagenese oder der genetischen Transformation des Stamms CNCM I-3689 umfasst.

4. Verfahren zum Erhalt einer Zellfraktion mit antimikrobiellen und/oder immunmodulatorischen Eigenschaften, **dadurch gekennzeichnet, dass** die Zellfraktion aus einem *Lactobacillus paracasei subsp. paracasei*-Stamm nach einem der Ansprüche 1 oder 2 erhalten wird.

5. Verfahren nach Anspruch 4, **dadurch gekennzeichnet, dass** die Zellfraktion ausgewählt ist aus der Gruppe bestehend aus: einer DNA-Präparation, einer Bakterienwandpräparation, einem Kulturüberstand und einer Fraktion dieses Überstands.

6. Zusammensetzung, umfassend einen *Lactobacillus paracasei subsp. paracasei*-Stamm nach einem der Ansprüche 1 oder 2 oder eine nach dem Verfahren gemäß einem der Ansprüche 4 oder 5 erhaltene Zellfraktion.

7. Zusammensetzung nach Anspruch 6, **dadurch gekennzeichnet, dass** es sich um ein Lebensmittelprodukt handelt.

8. Zusammensetzung nach Anspruch 6, **dadurch gekennzeichnet, dass** es sich um ein pharmazeutisches Produkt handelt.

9. Zusammensetzung nach Anspruch 6, **dadurch gekennzeichnet, dass** es sich um ein Kosmetikprodukt handelt.

10. Verwendung eines *Lactobacillus paracasei subsp. paracasei*-Stamms nach einem der Ansprüche 1 oder 2 oder einer nach dem Verfahren gemäß einem der Ansprüche 4 oder 5 erhaltenen Zellfraktion zum Erhalt einer Zusammensetzung, ausgewählt aus der Gruppe bestehend aus Lebensmittelprodukten, pharmazeutischen Produkten und Kosmetikprodukten.

11. *Lactobacillus paracasei subsp. paracasei*-Stamm nach einem der Ansprüche 1 oder 2 zur Verwendung als Immunmodulator.

12. *Lactobacillus paracasei subsp. paracasei*-Stamm nach einem der Ansprüche 1 oder 2 zur Verwendung als antimikrobielles Mittel.

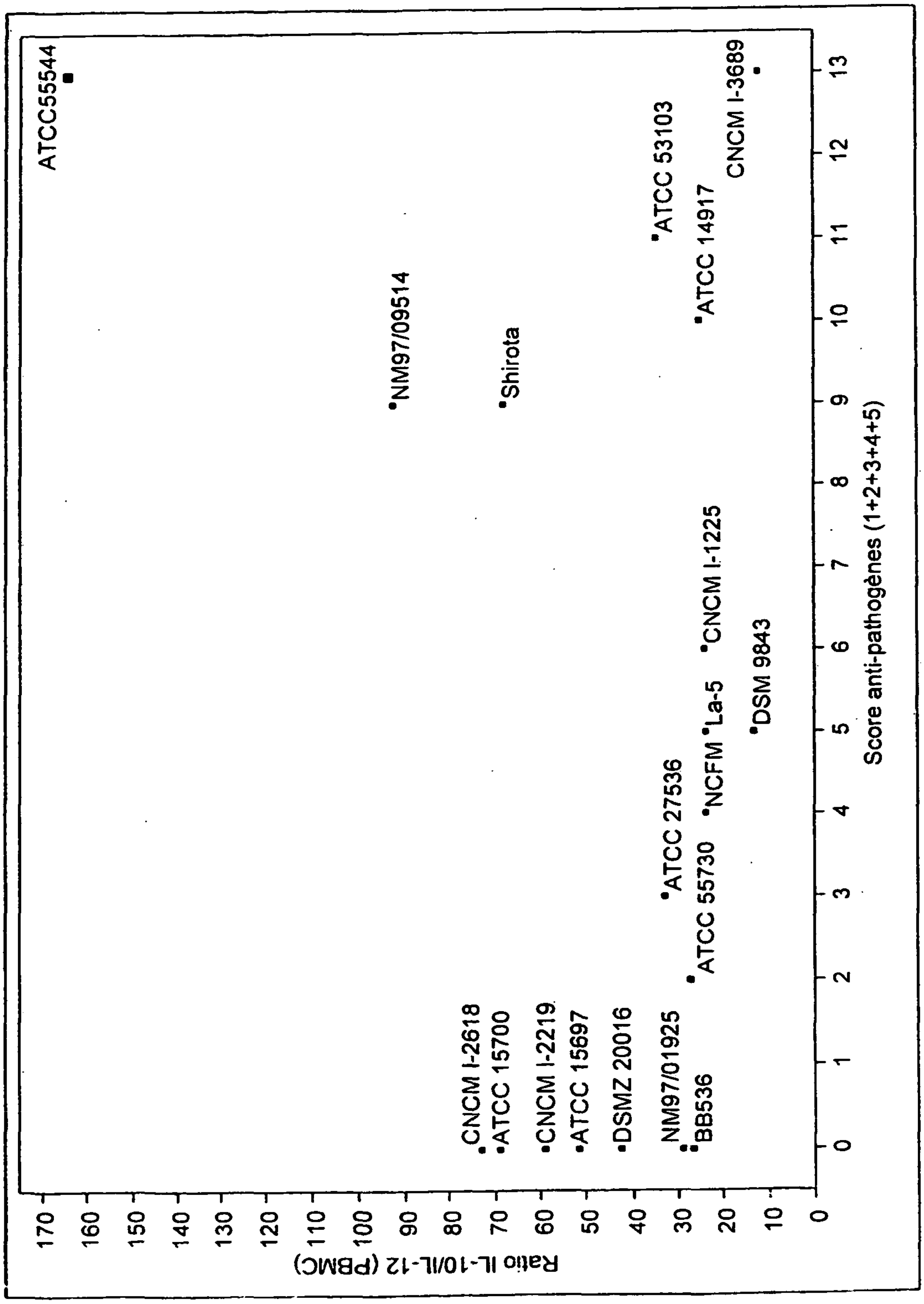
Ratio IL-10/IL-12 (PBMC)
Score anti-pathogènes (1+2+3+4+5)
ATCC55544
CNCM I-3689
ATCC 53103
ATCC 14917
NM97/09514
Shirota
CNCM I-1225
DSM 9843
La-5
NCFM
ATCC 27536
ATCC 55730
CNCM I-2618
ATCC 15700
CNCM I-2219
ATCC 15697
DSMZ 20016
NM97/01925
BB536

Figure 1

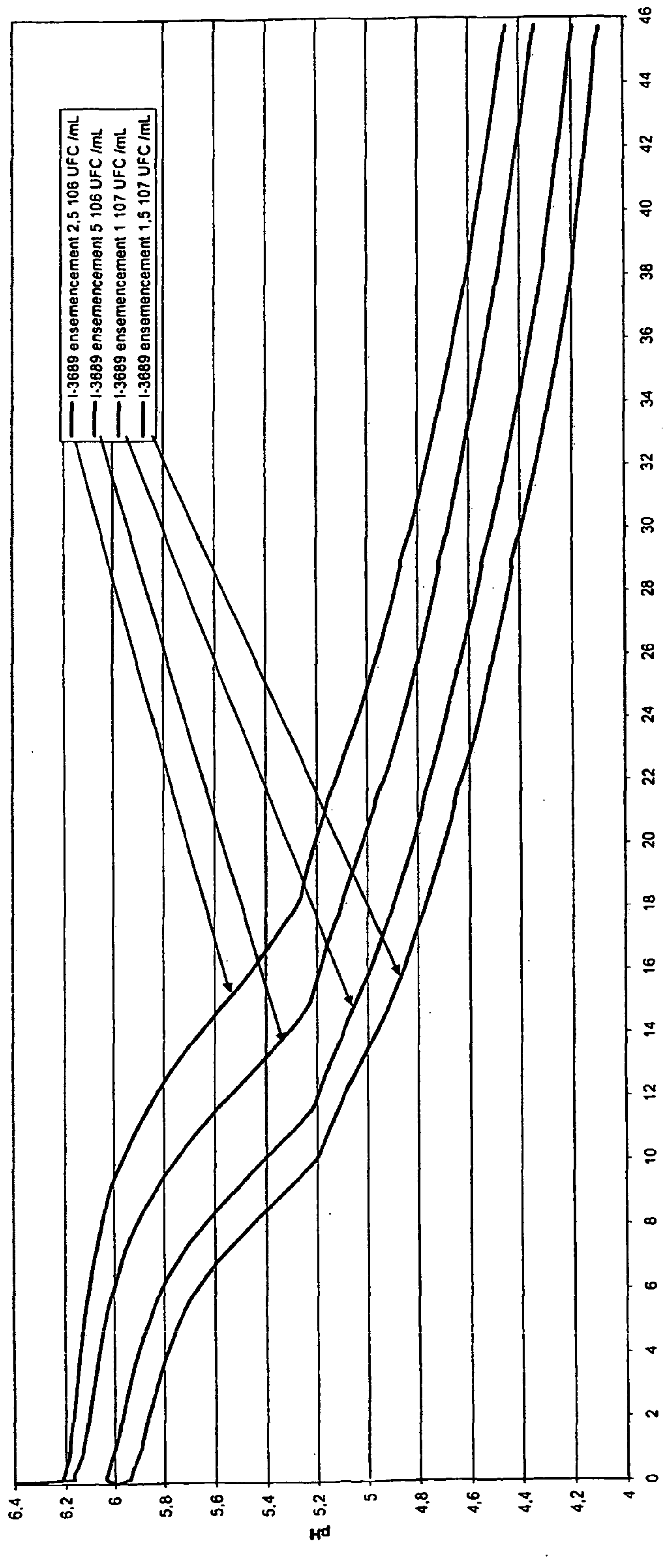
I-3689 ensemencement 2,5 106 UFC /mL
I-3689 ensemencement 5 106 UFC /mL
I-3689 ensemencement 1 107 UFC /mL
I-3689 ensemencement 1,5 107 UFC /mL
pH
6,4
6,2
6
5,8
5,6
5,4
5,2
5
4,8
4,6
4,4
4,2
4
Durée (h)
0
2
4
6
8
10
12
14
16
18
20
22
24
26
28
30
32
34
36
38
40
42
44
46

Figure 2

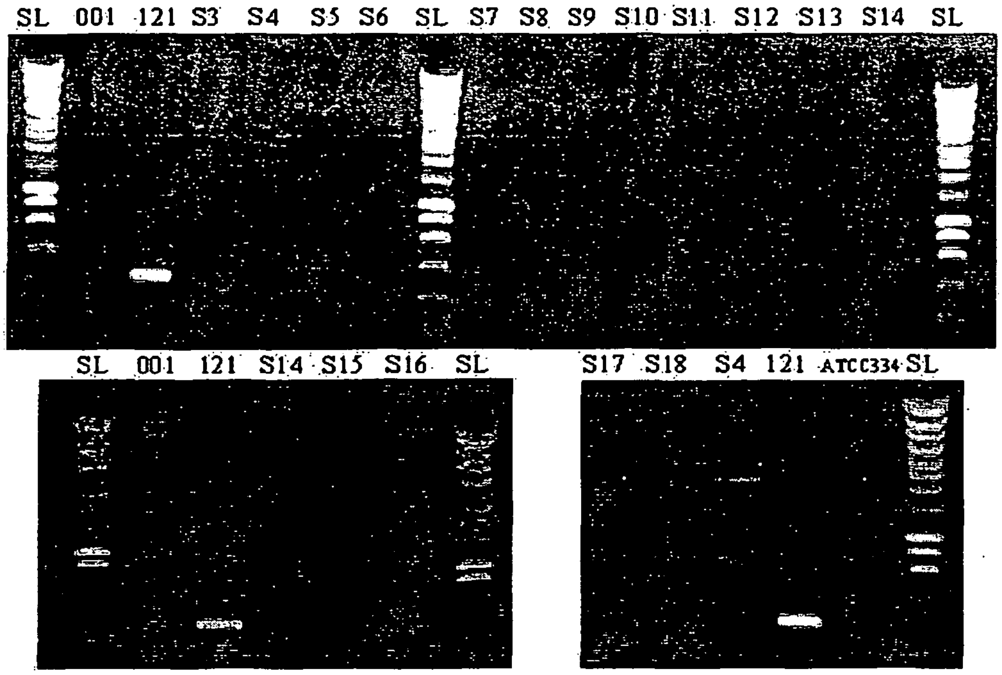
SL 001 121 S3 S4 S5 S6 SL S7 S8 S9 S10 S11 S12 S13 S14 SL
SL 001 121 S14 S15 S16 SL
S17 S18 S4 121 ATCC334 SL

Figure 3

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0794707 A
- WO 2004093898 A
- EP 0577903 A
- WO 2004032639 A
- US 4839281 A
- WO 9910476 A
- WO 9391823 A
- WO 2004034808 A
- EP 1189517 A


### Littérature non-brevet citée dans la description


- **GURY et al.** *Arch Microbiol.,* 2004, vol. 182, 337-45
- **VELEZ et al.** *Appl Environ Microbiol.,* 2007, vol. 73, 3595-3604
- **PATNAIK et al.** *Nat Biotechnol,* 2002, vol. 20, 707-12
- **WANG Y. et al.** *J Biotechnol.,* 2007, vol. 129, 510-15
- **ELLIKER et al.** *J. Dairy Sci.,* 1956, vol. 39, 1611-1612
- **DE MAN et al.** *J. Appl. Bacteriol.,* 1960, vol. 23, 130-135
- **JANSEN et al.** *OMICS,* 2002, vol. 6 (1), 23-33